(19) Europäisches Patentamt
European Patent Office
Office européen des brevets

(11) **EP 2 705 812 A1**

(12) **EUROPÄISCHE PATENTANMELDUNG**

(43) Veröffentlichungstag:
**12.03.2014 Patentblatt 2014/11**

(21) Anmeldenummer: **12183091.3**

(22) Anmeldetag: **05.09.2012**

(51) Int Cl.:
*A61F 9/008* (2006.01)          *H01S 3/00* (2006.01)
*B23K 26/06* (2014.01)          *B23K 26/38* (2014.01)
*B23K 26/40* (2014.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Universität zu Lübeck**
**23562 Lübeck (DE)**

(72) Erfinder:
• **Vogel, Alfred**
**23568 Lübeck (DE)**

• **Freidank, Sebastian**
**23568 Lübeck (DE)**
• **Linz, Norbert**
**23562 Lübeck (DE)**

(74) Vertreter: **Hansen, Jochen**
**Hansen und Heeschen**
**Patentanwälte**
**Eisenbahnstrasse 5**
**21680 Stade (DE)**

(54) **Vorrichtung zum Laserschneiden innerhalb transparenter Materialien**

(57)      Die Erfindung betrifft eine Vorrichtung zum Laserschneiden transparenter Materialien, die dazu ausgebildet ist, sowohl normales Laserlicht (z.B. Gaußscher Strahl) als auch Laserlicht mit helikalen Phasenfronten zu applizieren. Die Form des Laserfokus ist dadurch nach Wahl des Nutzers umschaltbar, beispielsweise zwischen einer Stab- und einer Scheibenform. Der Scheibenfokus eignet sich besonders zur Schnittführung entlang Linien oder Flächen, die im Wesentlichen senkrecht zur Einstrahlrichtung verlaufen. Dies gilt vor allem für lamellär geschichtete Strukturen.

Fig. 4 a)

Fig. 4 b)

**Beschreibung**

Die Erfindung betrifft eine Vorrichtung zum Laserschneiden eines transparenten Materials, die ausgebildet ist, das Laserlicht in eine Mehrzahl vorbestimmter Spots innerhalb des Material zu fokussieren, wobei die Spots auf einer vorbestimmten Schnittlinie oder -fläche liegen, die im Wesentlichen senkrecht zur Einstrahlrichtung des Laserlichts verläuft. Die Erfindung betrifft auch eine Vorrichtung zur Laserbearbeitung biologischen Gewebes, insbesondere des lebenden Auges.

Die Erzeugung feiner Effekte bei der Lasermaterialbearbeitung setzt die lokalisierte Deponierung geringer Energiemengen voraus. In für das Laserlicht transparenten Materialien wie z.B. Glas, Quarz, Wasser, unpigmentiertem Körpergewebe oder Zellen kann eine lokalisierte Energiedeponierung nur durch Mehrphotonenprozesse in der Form von Multiphotonenionisation und Lawinenionisation erfolgen, die zur Ausbildung eines Plasmas führen (quasifreie Ladungsträger im Material bestehend aus einer Mischung von Elektronen und Ionen). Da das Auftreten der Mehrphotonenprozesse nichtlinear von der Laserlichtintensität abhängt, spricht man von "nichtlinearer Absorption". Und da die Plasmabildungsrate oberhalb einer Schwelle, die von Material und Laserparametern abhängt, extrem stark zunimmt, wird der Plasmabildungsprozess in diesem Parameterbereich auch "optischer Durchbruch" genannt.

Höchst präzise Materialbearbeitung durch nichtlineare Absorption erfordert, dass räumlich lokalisiert reproduzierbar geringe Energiemengen in das Material eingetragen (deponiert) werden können. Die gute räumliche Lokalisation wird in erster Linie durch Fokussierung der Laserpulse mittels aberrationsfreier Optiken hoher numerischer Apertur erreicht.

Bei Dissektion innerhalb von transparenten Materialien ist oftmals eine Schnittführung erwünscht, die nicht entlang der Einstrahlrichtung des Laserstrahls erfolgt, sondern unter einem vorbestimmten Winkel zur Einstrahlrichtung. Häufig soll die Dissektion sogar senkrecht oder annähernd senkrecht zur Einstrahlrichtung erfolgen, um einen Schnitt zu erzeugen, der parallel oder annähernd parallel zur Oberfläche des transparenten Mediums ausgerichtet ist. Oft wird nicht nur eine Schnittlinie erzeugt, sondern die Schnitte werden zweidimensional entlang einer Schnittfläche ausgeführt, die im Wesentlichen parallel zur Oberfläche des Materials liegt, um eine Schicht des Materials abzutrennen. Dieser Fall tritt beispielsweise in der refraktiven Hornhautchirurgie (Laser-in-situ-Keratomileusis, kurz: LASIK) auf.

Für die eigentliche Schnittführung werden fokussierte Laserpulse nebeneinander in einem Punktraster appliziert. An jedem Rasterpunkt (i. F. auch: Spot) wird ein Plasma erzeugt, welches das transparente Material im Fokusvolumen desintegriert und bei hinreichend großer Energiedichte eine Mikroexplosion bewirkt, die einen Hohlraum (bzw. eine transiente Kavitationsblase in Flüssigkeiten oder biologischen Geweben) erzeugt.

Der jeweils wirksame Schneidemechanismus hängt vom Abstand der Rasterpunkte und von der Energiedichte der Plasmen ab. Die Energiedichte wird durch die Laserpulsenergie und die materialabhängige Schwelle für den optischen Durchbruch bestimmt, die ihrerseits von der Laserpulsdauer und der Laserwellenlänge sowie von der Laserstrahlqualität und dem Konvergenzwinkel (numerische Apertur) des fokussierten Laserstrahles abhängt. Bei ansonsten gleichen Bestrahlungsparametern ist die Plasmaenergiedichte in einem großen Bereich durch Variation der Laserpulsenergie einstellbar.

Die Schneidemechanismen lassen sich in zwei Kategorien unterteilen und werden gewöhnlich als Desintegrationsmodus und als Aufspaltungsmodus bezeichnet.

*Desintegrationsmodus:* Werden Laserplasmen geringer Energie- und Elektronendichte erzeugt, so bewirken die freien Elektronen durch Bindungsbrüche eine Desintegration des Materials in den Laserfoki. Dies ist mit Materialverdampfung oder Materialzersetzung verbunden. Die Materialauflösung in biologischen Geweben führt beispielsweise zur Entstehung von Gasblasen mit Lebensdauern im Bereich von Millisekunden bis Sekunden, die zwar nicht zum Schneideeffekt beitragen, aber als Indikator für die Materialauflösung dienen können. Bei Überlapp der Fokusvolumina und einer hinreichend großen Dichte von Bindungsbrüchen kann so ein zusammenhängender Schnitt erzeugt werden. Obwohl die Einzelpulsenergie gering ist, erfordert die Desintegration einer zusammenhängenden Materialschicht die akkumulierte Wirkung einer großen Zahl von Laserpulsen und daher eine relativ hohe Gesamtenergie.

*Aufspaltungsmodus*: Beim Aufspaltungsmodus wird der laterale Abstand D im Raster der Fokuspunkte größer gewählt als der Fokusdurchmesser d und beträgt üblicherweise etwa 6-10 Mikrometer. Hier kann ein zusammenhängender Schnitt nur durch die Mikroexplosionswirkung der Plasmen und das Zusammenwachsen der von den Plasmen erzeugten transienten Hohlräume (Kavitationsblasen) hergestellt werden. Für die Erzeugung der Hohlräume sind Plasmen höherer Energiedichte erforderlich als für den Desintegrationsmodus. In einem Material mit geschichteter Struktur (z.B. das Hornhautstroma des Auges) wird das Zusammenwachsen der Hohlräume zu einem Schnitt erleichtert. Der Schneidevorgang ist also ein durch die Expansion lasererzeugter Kavitationsblasen angetriebener Aufspaltungsprozess, der vorzugsweise entlang mechanischer Schwachstellen verläuft. Analogien hierzu sind das Aufspalten von Schieferplatten ausgehend von lokaler mechanischer Einwirkung oder das Spalten von astfreiem Holz entlang der Faserrichtung mit einem Beil. Obgleich die zur Erzeugung von Mikroexplosionen erforderliche Einzelpulsenergie im Aufspaltungsmodus größer ist als im Desintegrationsmodus, ist die erforderliche Gesamtenergie in der Regel geringer, weil nicht der Aggregatzustand einer zusammenhängenden Materialschicht geändert werden muss, sondern eine mechanische Trennung entlang von Schwachstellen des Materials erfolgt und somit eine geringere Anzahl an Einzelpulsen erforderlich ist. Der Materialverlust ist dementsprechend ebenfalls geringer als im Desintegrationsmodus.

Bei beiden Schneidemechanismen wird die Schnittpräzision maßgeblich durch die Länge des Laserplasmas bestimmt, die ihrerseits von der Länge des Laserfokus abhängt. Fokusdurchmesser d und Fokuslänge l sind bei voller Ausnutzung der Apertur des Schneideobjektivs gegeben durch:

$$d = 1{,}22 \frac{\lambda}{NA} \qquad (1)$$

$$l = 4\frac{d^2}{\lambda} \approx 6\frac{\lambda}{NA^2} \qquad (2)$$

Hierbei bezeichnen $\lambda$ die Wellenlänge des verwendeten Laserlichtes und NA die numerische Apertur des Schneideobjektives, die mit dem halben Öffnungswinkel $\alpha$ durch NA = n sin$\alpha$ verknüpft ist (n ist der Brechungsindex des Mediums, in welches das Licht fokussiert wird). An der Schwelle zur Plasmabildung ist die Plasmalänge ähnlich groß wie die Fokuslänge l und nimmt bei Erhöhung der Laserpulsenergie zu. Die Schneidepräzision wird durch die Lokalisierbarkeit des Schnittes in Strahlrichtung und die Schnittbreite definiert, welche beide mit der Plasmalänge verknüpft sind. Aus Gleichung (2) ist ersichtlich, dass die Schneidepräzision zunimmt, wenn die numerische Apertur erhöht oder die Wellenlänge $\lambda$ verringert wird.

Die gegenwärtig auf dem Markt für refraktive Hornhautchirurgie erhältlichen Femtosekunden-Lasersysteme arbeiten mit Wellenlängen im nahen Infrarot zwischen 1030 nm und 1060 nm. Bei diesen Systemen wird eine Erhöhung der Schneidepräzision vorwiegend durch eine Vergrößerung der NA angestrebt. Alternativ kommt auch die Verwendung ultravioletter Laserstrahlung in Betracht. Die gegenüber Infrarot-Femtosekunden-Pulsen auf ca. 1/3 verkürzte Wellenlänge führt dabei gemäß Gleichung (2) zu einer erheblichen Verbesserung der Schneidepräzision.

Die Druckschrift EP 1 787 607 A1 schlägt die Verwendung von gepulstem UV-Licht mit Wellenlängen zwischen etwa 190 nm und 380 nm für die LASIK-Behandlung vor, wobei die UV-Pulse mittels Frequenzvervielfachung aus Infrarot-Femtosekunden-Pulsen erzeugt werden und eine Pulsdauer bis hin zu maximal 10 ps aufweisen. Die Autoren der EP 1 787 607 A1 gehen davon aus, dass UV-Pulse mit Pulsenergien um etwa 10 nJ und Repetitionsraten zwischen 100 und 500 kHz einerseits zu präzisen Schnitten im Hornhautgewebe führen und andererseits nahezu vollständig in der Kornea oder der Linse absorbiert werden. Energieeinträge durch Resttransmissionen in die Retina werden als praktisch ausgeschlossen betrachtet. Es ist allerdings bemerkenswert, dass photochemische Veränderungen des Gewebes im Bereich der UV-Einstrahlung als ungewollte Nebenwirkung mit keinem Wort angesprochen werden.

Aus der Druckschrift DE 10 2007 028 042 B3 geht hervor, dass eine sehr feine Laserbearbeitung transparenter Materialien mit zeitlich glatten Ultraviolett-Nanosekunden-Pulsen möglich ist. Insbesondere Laserpulse mit 355 nm Wellenlänge und 0,7 ns Pulsdauer können zur Erzeugung von LASIK Flaps in der Hornhaut genutzt werden, wobei im Aufspaltungsmodus geschnitten wird (Vogel A, Linz N, Freidank S, Faust S, Schwed S (2011b) LASIK Flaperzeugung mit UV Subnanosekundenpulsen. Der Augenspiegel 12,2011: 32-35). Der Vorteil dieses Ansatzes liegt in der Möglichkeit der direkten Erzeugung des UV-Laserlichts mit einem faserverstärkten Mikrochiplaser, worin ein erhebliches Potenzial zur Kostensenkung zu sehen ist. Zudem sind bei Verwendung von Nanosekundenpulsen wegen der geringeren Spitzenleistungen nichtlineare Ausbreitungseffekte und Filamentierung besser vermeidbar als mit Femtosekundenpulsen.

Aus der US 2005/245915 A1 ist ein Verfahren zum Ausführen eines Schnitts entlang einer kuppelförmigen Schnittfläche im Hornhautstromagewebe des lebenden Auges mit einem Infrarot-Femtosekunden-Pulslaser zu entnehmen. Die Schnittfläche liegt im Wesentlichen parallel zur Hornhautoberfläche und zugleich im Wesentlichen senkrecht zur Einstrahlrichtung des Laserstrahls. Es wird ein Raster von Fokuspositionen auf der Schnittfläche vorbestimmt, wobei in jedem der Spots eine Blase vorbestimmten Durchmessers erzeugt werden soll. Der Laserfokusdurchmesser ist also kleiner oder gleich dem Blasendurchmesser. Die Spots sollen - senkrecht zur Einstrahlrichtung - Nächstnachbarabstände zueinander aufweisen, die in etwa dem Blasendurchmesser entsprechen. Soweit hieraus erkennbar, erfolgt der Laserschnitt im eingangs beschriebenen Aufspaltungsmodus, und nicht im Desintegrationsmodus.

Die Kombination eines Spotrasters gemäß z.B. der US 2005/245915 A1 mit einem ultravioletten Bearbeitungslaser nach der DE 10 2007 028 042 B3 zur Erzielung eines präziseren, insbesondere entlang der Einstrahlrichtung schärfer lokalisierten Flächenschnitts erweist sich zwar als erfolgreich gerade auch zur Bearbeitung biologischer Gewebe. Doch der deutlich reduzierte Fokusdurchmesser des UV-Lasers erfordert bei konstanter Einzelpulsenergie eine höhere Rasterpunktdichte, und somit ist die zu deponierende Gesamtenergie eines Flächenschnitts deutlich höher als bei der Verwendung eines Infrarot-Femtosekunden-Pulslasers. Speziell für lebendes Gewebe ergibt sich dadurch ein Dosisproblem, weil das Ausmaß möglicher UV-induzierter photochemischer Nebenwirkungen gemäß dem Reziprozitätsgesetz von Bunsen und Roscoe mit der Gesamt-Bestrahlungsdosis (J/cm$^2$) skaliert.

Hohe Schnittgeschwindigkeit und Schnittpräzision bei zugleich minimalen photomechanischen Nebenwirkungen sind generell konkurrierende Zielsetzungen, und bei Verwendung eines UV Lasers kommt noch die Minimierung photochemischer Nebenwirkungen hinzu. Eine hohe Schnittgeschwindigkeit erfordert neben einer hohen Laserpulsrepetitionsrate auch einen großen Rasterpunktabstand. Wird letzterer vergrößert, so begünstigt dies zugleich die Verringerung der gesamten Schneidenergie und vermeidet somit photochemische Effekte. Wird der Rasterpunktabstand jedoch zu groß gewählt, muss die Einzelpulsenergie so stark erhöht werden, dass die Schneidepräzision leidet und das Ausmaß möglicher Nebenwirkungen durch die mechanische Wirkung der lasererzeugten Stoßwellen und Kavitationsblasen deutlich zunimmt. Der gleiche nachteilige Effekt tritt auf, wenn bei konstantem Rasterpunktabstand der Fokusdurchmesser stark reduziert wird: nun muss die Einzelpulsenergie erhöht werden, um die Aufspaltungsdistanz auf das erforderliche Maß zu vergrößern.

Vor diesem Hintergrund kann man nach Verbesserungsmöglichkeiten suchen, die auf die Formung des Laserfokus bzw. des erzeugten Plasmas abzielen.

Der Stand der Technik kennt bereits Verfahren zur Fokusformung für die Lasermaterialbearbeitung in transparenten Medien. Sie zielen in der Regel auf eine Fokusverlängerung ab, falls möglich sogar mit einer Verminderung des lateralen Durchmessers: Beispielsweise wird ein Bessel-Strahl anstelle eines Gaußschen Strahls verwendet, um einen gegenüber dem Gaußschen Strahl stark verlängerten Fokusbereich zu erzeugen (McGloin D, Dholakia K (2005) Bessel beams: Diffraction in a new light. Contemp. Phys. 46:15-28). Bessel-Strahlen eignen sich insofern für die Optimierung von Schnittführungen in Einstrahlrichtung des Lasers, wie etwa beim Randschnitt bei der LASIK-FlapErzeugung.

Zusätzliche Möglichkeiten zur Erhöhung der Plasmalänge ergeben sich durch nichtlineare Strahlausbreitung (Selbstfokussierung durch das erzeugte Plasma) und Filamentierung in transparenten Medien. Diese werden zur Erzeugung von elongierten Lasereffekten, Bohrlöchern und Kanälen mit großem Aspektverhältnis genutzt (Ashkenasi D, Varel H, Rosenfeld A, Henz S, Herrmann J, Cambell EEB (1998) Application of self-focusing of ps laser pulses for three-dimensional microstructuring of transparent materials. Appl. Phys. Lett. 72: 1442-1444).

Verwendet man Laserpulse hoher Leistung und verläuft die Schnittrichtung im Wesentlichen senkrecht zur Laserstrahlrichtung, dann soll eine Fokusverlängerung durch nichtlineare Strahlausbreitung und Filamentierung oftmals möglichst vermieden werden. Störungen der Schnittführung durch Fokusverlängerung wurden für Femtosekunden LASIK beschrieben (Arnold CL, Heisterkamp A. Ertmer W, Lubatschowski H (2004) Streak formation as side effect of optical breakdown during processing the bulk of transparent Kerr media with ultra-short laser pulses. Appl. Phys. B 80:247-253).

So genannte Vortex Beams haben bei gleichem Fokussierungswinkel einen größeren Fokusquerschnitt als Gaußsche Strahlen. Dadurch weisen sie eine mindestens 4-fach höhere Selbstfokussierungsschwelle auf als Gaußsche Strahlen (Vuong LT et al. (2006) Collapse of optical vortices. Phys. Rev. Lett 96:133901(4pp)). Der Name "Vortex Beam" rührt daher, dass die Intensitätsverteilung auf der optischen Achse eine Nullstelle aufweist und man diese Singularität als Wirbel im elektromagnetischen Feld verstehen kann. Laguerre-Gaußsche (LG) Ausbreitungsmoden des Lichtes haben diese Eigenschaft, wobei der Grundmode mit LG (0,1) bezeichnet wird (Yao and Padgett, "Orbital angular momentum: origins, behavior and applications", Advances in Optics and Photonics 3, 161-204 (2011)).

Die LG-Moden bilden einen vollständigen Satz von Moden, nach denen Licht mit helikalen Phasenfronten ("helically phased beams") entwickelt werden kann. Die einzelnen helikalen Photonen tragen dabei Drehimpulse. Helikales Laserlicht kann aus nicht-helikalem Laserlicht (üblich: Gaußscher Strahl, aber nicht darauf beschränkt) erzeugt werden, wenn man den nicht-helikalen Strahl durch eine helikale Phasenplatte ("spiral phase plate"), ein speziell ausgebildetes, computergeneriertes Beugungsgitter ("diffractive optical element", oft auch als "subwavelength structure" bezeichnet), einen elektronisch ansteuerbaren räumlichen Lichtmodulator ("spatial light modulator") oder eine Zylinderlinsenanordnung transmittiert (Yao and Padgett, "Orbital angular momentum: origins, behavior and applications", Advances in Optics and Photonics 3, 161-204 (2011)). Die vorgenannten Mittel zur Modenkonversion von nicht-helikalem in helikales Laserlicht sind jedoch entweder schwer herstellbar, nur für limitierte Laserleistung geeignet (z.B. Phasenplatten aus Kunststoff und Beugungsgitter ausgeführt als strukturierte Metallisierung oder als Matrix aus Flüssigkristallen) oder stellen Anforderungen an das nicht-helikale Licht, die von herkömmlichen Laserlichtquellen für die Materialbearbeitung nicht erfüllt werden (z.B. das Auftreten bestimmter höherer Gaußscher Moden).

Eine effiziente Modenkonversion für Hochleistungslaseranwendungen kann indes durch helikale Phasenplatten aus Quarzglas oder einem anderen lichtbeständigen doppelbrechenden Material erreicht werden (Machavariani G, Lumer Y, Moshe I, Meir A, Jackel S (2007) Efficient extracavity generation of radially and azimuthally polarized laser beams. Opt. Lett. 32: 1468-1470).

Eine ideale Konversion in den Laguerre-Gaußschen Grundmode LG (0,1) würde durch ein optisches Bauteil erreicht, das einen Phasenhub erzeugt, der bei Drehung um die optische Achse um 360 Grad kontinuierlich von Null auf $2\pi$ ansteigt. Solche Bauteile mit kontinuierlichem Phasenhub lassen sich aber noch nicht aus einem Material herstellen, das sehr hohen Laserleistungen standhält.

Der Idealfall wird daher durch die Verwendung von segmentierten Phasenplatten angenähert, wobei die Näherung umso besser und mit umso höherer Effizienz erfolgt, je höher die Anzahl der Segmente ist. Durch eine nachgeschaltete Raumfrequenzfilterung lässt sich das an den Sektorgrenzen gestreute Licht eliminieren und eine fokale Intensitätsverteilung erzeugen, die dem idealen Laguerre-Gauss (0,1) Mode sehr nahe kommt (Machavariani et al. 2007).

Es ist die Aufgabe der Erfindung, eine Vorrichtung zum Laserschneiden transparenten Material dahingehend fortzubilden, dass sie das präzise Ausführen von Laserschnitten entlang einer Schnittlinie oder Schnittfläche, die im Wesentlichen senkrecht zur Einstrahlrichtung des Laserlichts verläuft, mit weniger Rasterpunkten oder geringerer Gesamtenergiedosis als im Stand der Technik erforderlich erreicht und dadurch photochemische und photomechanische Nebenwirkungen vermeidet.

Die Aufgabe wird gelöst durch eine Vorrichtung zum Laserschneiden eines transparenten Materials, die ausgebildet ist, das Laserlicht in eine Mehrzahl vorbestimmter Spots innerhalb des Materials zu fokussieren, wobei die Spots auf einer vorbestimmten Schnittlinie oder -fläche liegen, die im Wesentlichen senkrecht zur Einstrahlrichtung des Laserlichts verläuft, dadurch gekennzeichnet, dass die Vorrichtung in den Strahlengang des Laserlichts ein- und ausbringbare Mittel zur Modenkonversion in Laserlicht mit helikaler Phasenfront aufweist.

Die Unteransprüche geben vorteilhafte Ausgestaltungen an.

Nach bestem Wissen der Erfinder wurde bislang noch nicht vorgeschlagen, Laserlicht mit helikalen Phasenfronten (i. F. kurz: helikales Laserlicht) zur Ausführung von Schnitten in transparentem Material zu verwenden. Erst recht ist den Erfindern kein Vorschlag bekannt, helikales Laserlicht in der medizinischen Therapie, insbesondere in der Laserbehandlung des Auges einzusetzen.

Helikales Laserlicht besitzt immer einen optischen Wirbel, ist also ein typischer Vortex Beam. Zu seinen für die Erfindung als wesentlich erkannten Eigenschaften zählt, dass die Fokussierung des helikalen Laserstrahls auf eine torusförmige Lichtintensitätsverteilung und folglich auf einen ebenfalls torusförmigen Bereich der Plasmaerzeugung führt, der größer ist als der Fokusquerschnitt eines Gaußschen Strahls.

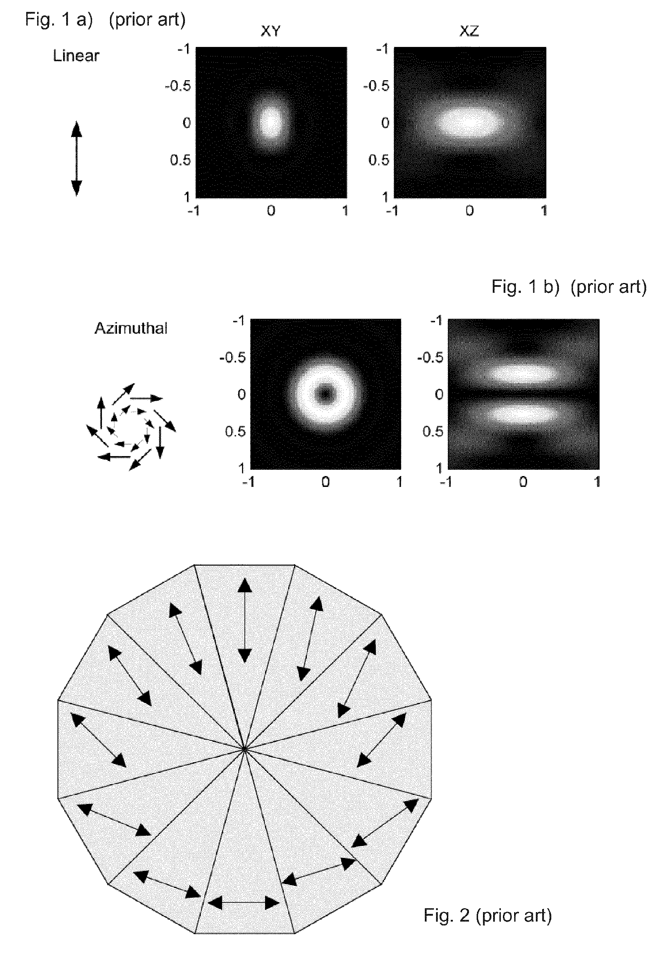

Ein Beispiel für die Intensitätsverteilung im Fokusbereich eines azimutal polarisierten Laserstrahls (ein Beispiel für helikales Laserlicht) im Vergleich mit dem Fokusbereich eines linear polarisierten Laserstrahls ist der Fig. 1 zu entnehmen (aus Hao X, Kuang C, Wang T, LiuX (2010) Effects of polarization on the de-excitation dark focal spot in STED microscopy. J. Opt. 12:115707 (8pp)). Erkennbar ist der Fokusdurchmesser gegenüber dem Wert beim linear polarisierten Strahl um etwa einen Faktor 2-3 vergrößert, wohingegen die Fokuslänge im Wesentlichen unverändert bleibt.

Der Grundgedanke der Erfindung ist, dass Vortex Beams dazu geeignet sind, einen gegenüber einem linear polarisierten Gauß-Strahl wenigstens verdoppelten Fokusdurchmesser bei etwa gleicher Fokuslänge zu erzeugen und somit einen größeren Spotabstand zu gestatten, ohne dass die Einzelpulsenergie erhöht werden muss. Dies steigert die Schnittgeschwindigkeit und vermeidet nachteilige mechanische Nebenwirkungen sowie - im Falle eines UV-Laserstrahls zur Bearbeitung lebenden Gewebes - das Überschreiten einer tolerierbaren Strahlungsdosis.

Bekanntlich ist es möglich, optische Elemente herzustellen, die in den Strahlengang eines herkömmlichen linear polarisierten Gauß-Strahles eingebracht zur Ausbildung eines Vortex Beams (z.B. mit azimutaler Polarisation) führen. Solche optischen Elemente, die im Folgenden als Mittel zur Modenkonversion in helikales Laserlicht (oder kurz: Mittel zur Modenkonversion) bezeichnet werden, sind erfindungsgemäß so in der Vorrichtung anzuordnen, dass sie in den Strahlengang des Lasers nach Bedarf einbringbar und ausbringbar sind. Auf diese Weise stehen dem Laserschneidsystem mehrere Fokusformen nach Wahl des Nutzers zur Verfügung.

Der herkömmliche Bearbeitungslaserstrahl (z.B. ein Gauß-Strahl) kann somit während des Einsatzes - vorzugsweise durch Einschwenken einer segmentierten Phasenplatte - sehr leicht in einen Vortex Beam umgewandelt werden, was unmittelbar wenigstens zur Verdopplung des Laserfokusdurchmessers führt. Mit den nunmehr verbreiterten Foki kann die Materialzersetzung in den vorbestimmten Rasterpunkten einer Schnittlinie oder Schnittfläche erfolgen, die im Wesentlichen senkrecht zur Einstrahlrichtung, also im Wesentlichen entlang der Richtung der erzielten Fokusverbreiterung, liegt. Nach der Ausführung des Laserschnitts kann der Vortex Beam - jetzt durch Ausschwenken der Phasenplatte - wieder in den Normalstrahl überführt werden.

Alternativ ist es möglich, den Bearbeitungslaserstrahl über wenigstens einen ansteuerbaren Ablenkspiegel abzulenken, wobei der wenigstens eine Ablenkspiegel in Abhängigkeit von seiner Ansteuerung den Strahlengang des Laserlichts durch die Mittel zur Modenkonversion führt oder an diesen vorbei.

Die erfindungsgemäß in den Strahlengang ein- und ausbringbaren Mittel zur Modenkonversion begünstigen die Ausführbarkeit von Schnitten im Wesentlichen senkrecht zur Einstrahlrichtung für jede Laserwellenlänge schon dadurch, dass durch die Vergrößerung des Fokusdurchmessers sowohl im Desintegrationsmodus als auch im Aufspaltungsmodus weniger Rasterpunkte zur Schnittausführung benötigt werden, was die Schnittgeschwindigkeit grundsätzlich erhöht.

Sehr vorteilhaft ist die Erfindung für UV-Laser, ganz besonders, wenn diese zum Schneiden in lebendem Gewebe eingesetzt werden. Besonders bevorzugt können mit der Erfindung auch Laserbearbeitungen am lebenden Auge mit Wellenlängen zwischen 300 nm und 400 nm erfolgen, wobei zugleich die Strahlungsdosis entweder optimiert, zumindest aber unter einem vorgegebenen Schwellenwert gehalten werden kann.

Eine weitere vorteilhafte Anwendung der Erfindung besteht in der Unterdrückung einer möglichen Fokusverlängerung durch nichtlineare Strahlausbreitung bei LASIK Flaperzeugung und bei der Linsenfragmentation mit ultrakurzen IR Laserpulsen. Für die Linsenzertrümmerung werden vergleichsweise schwach fokussierte energiereiche Laserpulse eingesetzt, so dass hier eine besonders hohe Neigung zur Filamentierung besteht. Die mindestens vierfache Erhöhung der Selbstfokussierungsschwelle beim Einsatz von Vortex Beams führt zu einer besseren Lokalisierbarkeit der Energiedeponierung in axialer Richtung und somit zu einem Schutz der Linsenkapsel und zur Vermeidung von lokalen Maxima der Strahlungsdosis auf der Netzhaut.

Es ist ferner eine vorteilhafte Ausgestaltung der erfindungsgemäßen Vorrichtung, wenn diese eine Einrichtung zur Ermittlung der Bestrahlungsdosis pro Flächeneinheit aufweist, die die Bestrahlungsparameter der Laserlichtquelle und die vorbestimmten Spotpositionen erfasst, daraus einen Dosiswert errechnet und diesen ausgibt. In einer weiterhin bevorzugten Ausgestaltung der Vorrichtung ist die Einrichtung zur Ermittlung der Bestrahlungsdosis pro Flächeneinheit dazu ausgebildet, bei Errechnen eines Dosiswertes, der einen vorgegebenen Schwellenwert übersteigt, die Bestrahlungsparameter der Laserlichtquelle selbsttätig zu ändern und/oder Spotpositionen mit größerem Spotabstand vorzubestimmen. Eine solche Einrichtung kann ein programmierbarer Mikroprozessor sein, vorzugsweise ein PC, der mit Schnittstellen zum Datenaustausch mit der Steuereinrichtung des Scanners und ggf. mit der Laserlichtquelle ausgestattet ist. Es ist gängig, dass ein einzelnes Computersystem die Funktionen aller Komponenten eines Laserschneidsystems überwacht und steuert. In einem solchen Computersystem ist eine Einrichtung zur Ermittlung der Bestrahlungsdosis in der Gestalt einer Softwareimplementation realisierbar.

Es soll an dieser Stelle betont werden, dass die Erfindung zwar ein breiteres Anwendungsspektrum besitzt, aber ihre Verwendung besonders vorteilhaft bei der Laser-in-situ-Keratomileusis (LASIK), flaploser Refraktionskorrektur durch Ausschneiden eines Lentikels, der lamellären Keratoplastik und/oder der Laser-Kataraktchirurgie ist. Überdies kann sie auch als ein Schlüsselkonzept zur Einführung von UV-Lasersystemen in die refraktive Augenchirurgie angesehen werden, weil sie eine Lösung der Dosisproblematik bietet.

Nachfolgend wird die Erfindung auch anhand der beiliegenden Zeichnungen genauer erläutert.

Darin zeigen:

Fig. 1      der Vergleich der Fokusformen von linear (oben) (a) und azimutal (unten) (b) polarisierter Laserstrahlung;

Fig. 2      die Skizze einer segmentierten Phasenplatte, die als ein mögliches Mittel zur Modenkonversion in den Strahlengang des Lasers eingebracht werden kann;

Fig. 3      Messwerte für Laserenergien bei der erfolgreichen Schnittführung mit einem UV-Lasersystem bei enukleirten Schweineaugen aufgetragen gegenüber dem verwendeten Spotabstand, insbesondere die Einzelpulsenergie (oben) (a) und die Gesamtdosis (unten) (b);

Fig. 4      eine Skizze der Aufspaltung eines lamellär geschichteten Materials mittels Laserdeposition in a) einen stabförmigen und b) einen scheibenförmigen Laserfokus;

Fig. 5      eine schematische Darstellung der erfindungsgemäßen Vorrichtung mit a) einschwenkbaren Mitteln zur Modenkonversion und b) ansteuerbaren Ablenkspiegeln zur Auswahl eines Strahlengangs.

Wie bereits erläutert, zieht das Einbringen eines Mittels zur Modenkonversion in einen linear polarisierten Laserstrahl mit z.B. Gaußschem Strahlprofil nach sich, dass ein Vortex Beam (hier: mit azimutaler Polarisation) entsteht. Wird dieser in ein transparentes Material fokussiert, so bildet sich ein toroidales Fokusvolumen wie in Fig. 1 unten dargestellt aus. Die Einstrahlrichtung liegt hier entlang der z-Achse. Zum Vergleich ist der Fokus des Lasers ohne das in den Strahl gebrachte optische Element oben in Fig. 1 dargestellt (alle Abbildungen sind Hao et al. (2010) entnommen). Die beiden rechten Bilder zeigen die xz-Ebenen und somit die Fokuslängen entlang der z-Richtung, die sich bei Gaußschem und bei Vortex Beam nur unwesentlich unterscheiden. Aus den beiden linken Bildern ist hingegen in der xy-Ebene senkrecht zur Einstrahlrichtung gut zu sehen, dass sich der Fokusdurchmesser beim Vortex Beam mehr als verdoppelt. Die Singularität im Zentrum des Vortex Beam hat für die Schneidwirkung gewöhnlich keine Bedeutung. Das Material wird dort entweder auch zersetzt oder zumindest aufgespalten, so dass das Material in einem diskus- oder scheibenförmigen Volumen zerstört wird. Daraus begründet sich, dass man auch von einem scheibenförmigen Laserfokus sprechen kann.

Ein scheibenförmiger Fokus kann sehr einfach durch das Einbringen eines optischen Elements erzeugt werden. Beispielsweise kann eine segmentierte Phasenplatte, bevorzugt aus doppelbrechendem Quarzglas mit jeweils unterschiedlicher Orientierung der kristalloptischen Achse in den einzelnen Segmenten, verwendet werden (Fig. 2). Die Orientierung der langsamen Lichtausbreitungsrichtung im Kristall ist jeweils durch die Pfeilrichtung in Fig. 2 angezeigt.

Dabei ist anzumerken, dass die einzelnen Segmente miteinander verklebt sind, und der Kleber unter Einwirkung von UV-Licht degradieren könnte. Segmentierte Phasenplatten für kurzgepulste UV Laserstrahlung sehr hoher Leistung befinden sich daher noch in der Entwicklung. Erste Tests zur UV-Beständigkeit im Labor der Erfinder sind jedoch erfolgversprechend verlaufen:

Für eine sichtbare Schädigung in der Kleberschicht durch UV Degradation wurde eine Schadensschwelle von etwa $3 \times 10^6$ (3 Millionen) $J/cm^2$ ermittelt. Nimmt man an, dass bei der geplanten Anwendung eine homogene Ausleuchtung einer Fläche des Modenkonverters von 10 mm Durchmesser mit Einzelpulsen von etwa 10 $\mu$J erfolgt, so könnten bis zum Erreichen dieser Schwelle etwa $2.4 \times 10^{11}$ Pulse appliziert werden, was bei einer Repetitionsrate von 150 kHz einer Lebenszeit von knapp 440 h entsprechen würde.

Bei einem klinischen Gerät zur LASIK Flaperzeugung kann durch einen Shutter am Laserausgang gewährleistet werden, dass die Beanspruchungszeit der Phasenplatte mit der Bestrahlungszeit des Auges identisch ist. Da die durchschnittliche Schnittzeit pro Auge unter 30 s liegt, könnte man innerhalb der erwarteten Lebensdauer der Phasenplatte 52800 Augen behandeln. Bei einer Behandlungsdauer pro Patient von etwa 15 min für beide Augen müsste die Phasenplatte erst nach etwa 5 Jahren ausgetauscht werden.

Es ist nach Kenntnis der Erfinder bislang nicht vorgeschlagen worden, eine Vorrichtung zum Laserschneiden in transparentem Material mit einem durch helikales Laserlicht absichtlich verbreiterten Fokus auszustatten. Auf den ersten Blick liegt dies dem Fachmann auch nicht nahe, weil die typischerweise gewünschte laterale Schnittpräzision verschlechtert wird. Bei der Schnittführung entlang von Linien oder Flächen, die im Wesentlichen senkrecht zur Einstrahlrichtung orientiert sind, kommt es aber nicht so sehr auf die laterale, sondern vorwiegend auf die axiale Schnittpräzision an. Letztere wird durch die Erfindung sogar verbessert, weil durch die Verringerung der zu überwindenden Aufspaltungsdistanz die Schnittführung besser im Bereich der Schneideebene lokalisiert bleibt.

Nachfolgende Überlegungen sollen die Vorteile verdeutlichen, die durch helikales Laserlicht und die damit erzeugbaren scheibenförmigen Laserfoki bezüglich der Gesamtbestrahlungsdosis und der Schneidepräzision erreicht werden:

Beim Schneiden im Desintegrationsmodus ist der Spotabstand $D$ kleiner als der Fokusdurchmesser $d$, wohingegen er im Aufspaltungsmodus größer ist. Beim Übergang zwischen Schneiden im Desintegrationsmodus und Schneiden im Aufspaltungsmodus entspricht der Radius der bei Applikation eines Laserpulses erzeugten Schnittfläche also gerade dem halben Fokusdurchmesser $d/2$. Für einen Fokusabstand $D > d$ muss jeweils die Distanz $(D/2 - d/2) =$

$(D-d)/2$ durch Aufspaltung überwunden werden. Die dazu erforderliche mechanische Arbeit wird durch die Energie der laserinduzierten Stoßwelle und Kavitationsblase aufgebracht. Die zum Schneiden eines Flächenelementes erforderliche Laserenergie $E_{ges}$ hängt dabei von $D$ und der Einzelpulsenergie $E_L$ ab. Bei konstanter Pulsenergie $E_L$ würde gelten

$$E_{ges} \propto \frac{1}{D^2}, \qquad (3)$$

weil die Zahl der Laserpulse pro Flächenelement mit $D$ quadratisch abnimmt. Tatsächlich ist die Pulsenergie aber keine Konstante, sondern muss so gewählt werden, dass sie eine Blase erzeugt, deren Größe ausreicht, um den Abstand zwischen benachbarten Rasterpunkten zuverlässig durch Aufspaltung zu überbrücken. Die mögliche Reichweite der Aufspaltung kann durch den Maximalradius $R_{max}$ der Blasen abgeschätzt werden, wobei dieser für eine zuverlässige Aufspaltung immer um einen bestimmten Faktor $k > 1$ größer sein sollte als der halbe Rasterpunktabstand:

$$R_{max} = k \times D/2 \qquad (4)$$

Der Stand der Technik kennt folgenden Zusammenhang zwischen der Laserpulsenergie $E_L$ und dem Maximalradius von in Wasser erzeugten Kavitationsblasen:

$$R_{max} \propto \left(E_L - E_c\right)^{1/3}, \quad (5a)$$

wobei $E_c$ der Energiebetrag ist, der während des Laserpulses durch den Fokus transmittiert wird, bevor die Plasmabildungsschwelle erreicht ist. In Schwellennähe nimmt (5a) zufolge $R_{max}$ zunächst rasch mit $E_L$ zu, doch für Pulsenergien deutlich oberhalb der Plasmabildungsschwelle verlangsamt sich die Zunahme, denn es gilt $E_L \gg E_c$ und somit näherungsweise

$$R_{max} \propto E_L^{1/3}. \qquad (5b)$$

Zwar sind die Details der Schneidedynamik im Hornhautgewebe noch wenig erforscht, doch weist die schwache Abhängigkeit zwischen $R_{max}$ und $E_L$ in Gleichung (5b) bereits darauf hin, dass bei großem Rasterpunktabstand $D \gg d$ eine deutliche Zunahme der Einzelpulsenergie erforderlich sein wird, um eine wachsende Distanz $(D-d)/2$ durch Aufspaltung zu überwinden. Im Extremfall, wenn der Fokusdurchmesser $d$ gegenüber dem Spotabstand $D$ vernachlässigbar klein wird, gilt schließlich mit den Gleichungen (4) und (5b):

$$E_L \propto D^3 \qquad\qquad (6)$$

Eine Vergrößerung von $D$ führt bei kleinen Werten der Rasterpunktabstände wegen der verringerten Pulszahl pro Flächenelement zunächst zu einer deutlichen Verminderung der für das Schneiden erforderlichen Gesamtenergie, d.h. Gl. (3) dominiert die Abhängigkeit $E_{\text{ges}}(D)$. Bei größeren Rasterpunktabständen kompensiert die Zunahme der erforderlichen Einzelpulsenergie weitgehend den Effekt der verringerten Pulszahl. Bei noch größeren Rasterpunktabständen wird erwartet, dass die Zunahme der erforderlichen Einzelpulsenergie gemäß Gl. (6) schließlich den Effekt der verringerten Pulszahl gemäß Gl. (3) dominiert und die erforderliche Gesamtschneideenergie sogar mit $D$ zunimmt.

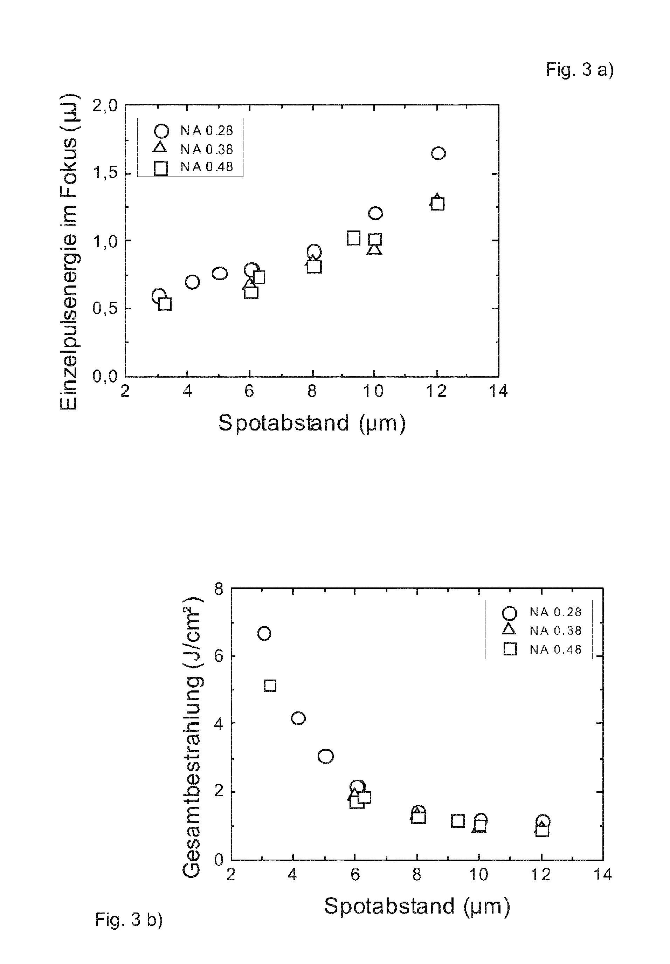

Experimente an enukleierten Schweineaugen verdeutlichen die vorgenannte Situation. Fig. 3 zeigt für mehrere erfolgreich ausgeführte Laserdissektionen bei verschiedenen numerischen Aperturen (NA) die erforderlichen Einzelpulsenergien (oben) und die Gesamtbestrahlungsdosen (unten) jeweils in Abhängigkeit vom Spotabstand. Während beim Übergang von 3 μm zu 12 μm Spotabstand die benötigte Pulsenergie etwa verdreifacht werden muss, lässt sich doch zugleich die Gesamtstrahlungsdosis ungefähr auf ein Sechstel verringern. Man erkennt aber auch bereits, dass sich die Gesamtdosis durch eine weitere Vergrößerung des Spotabstandes unter gleichzeitiger Erhöhung der Pulsenergie nicht mehr verringern lässt. Es wäre dann aber mit stärkeren Nebenwirkungen in der Umgebung der Spots zu rechnen.

Die durch die erfindungsgemäße Vorrichtung mögliche Vergrößerung des Fokusdurchmessers d bei konstantem Rasterpunktabstand D vermindert nun die zu überwindende Aufspaltungsdistanz (D-d)/2 und reduziert somit die erforderliche mechanische Schneidearbeit, die durch Stoßwelle und Kavitationsblase aufgebracht werden muss. Wird der Fokusdurchmesser beispielsweise von 1 μm auf 3 μm vergrößert, so erhöht sich die durch Desintegration im Plasma geschnittene Fläche auf das Neunfache. Bei 6 μm Rasterpunktabstand entspricht dies einer deutlichen Erhöhung des durch Desintegration geschnittenen Flächenanteils. Er steigt von lediglich etwa 1/46 auf knapp 1/5 an.

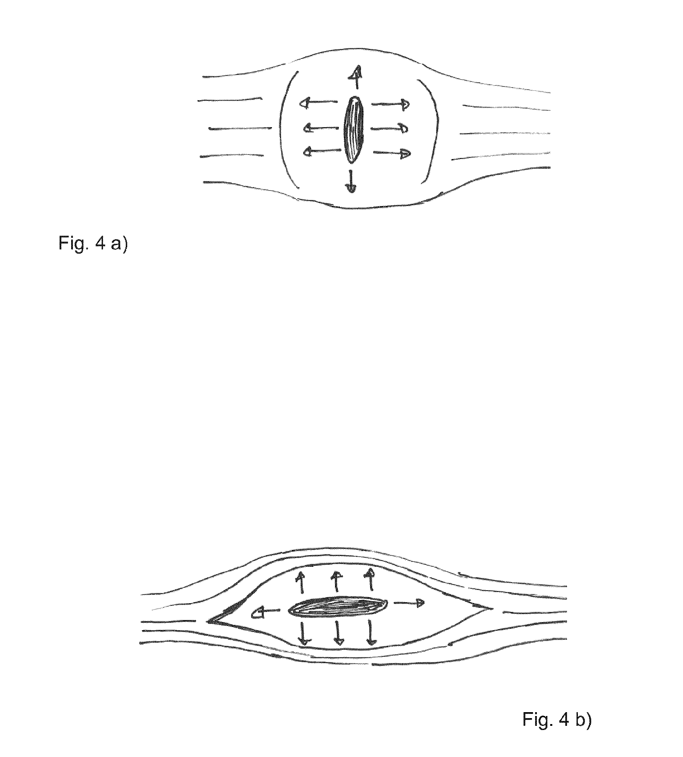

Ein scheibenförmiger Fokus unterstützt insbesondere in einer lamellär geschichteten Struktur wie der Hornhaut eine Aufspaltung entlang der Lamellenrichtung, weil durch die Kräfteverteilung bei der Plasma- und Blasenexpansion die Lamellen auseinandergeschoben werden und durch die Ausrichtung der Fokusscheibe eine Vorzugsrichtung und Vorzugsebene für die Aufspaltung vorgegeben werden. Die Skizze der Fig. 4 soll die Aufspaltung von Hornhautlamellen nach Energiedeponierung a) in einen scheibenförmigen Laserfokus und b) in einen stabförmigen Fokus darstellen. Dabei deuten die Pfeile die Richtungen der von den Foki ausgehenden Kraftwirkungen an. Man sieht leicht ein, dass der scheibenförmige Fokus das Ablösen der Lamellen voneinander besser unterstützt als der stabförmige Fokus. Die laterale Aufspaltung des Materials durch das Applizieren helikaler Laserpulse hat daher für jeden einzelnen Spot eine größere Reichweite und erlaubt somit das Schneiden mit weniger Rasterpunkten. Zudem verbessert sich die axiale Schneidepräzision, weil durch die Verringerung der zu überwindenden Aufspaltungsdistanz die Schnittführung besser im Bereich der Schnittebene lokalisiert bleibt.

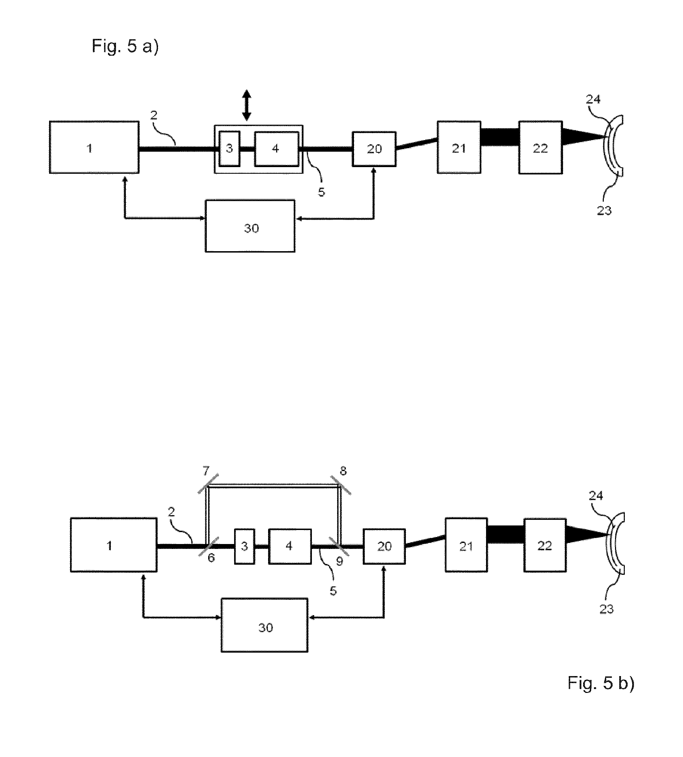

Abschließend werden zwei Ausführungsbeispiele der erfindungsgemäßen Vorrichtung zum Laserschneiden in Fig. 5 skizziert.

In Fig. 5a) und b) ist jeweils ein Laser 1 dargestellt, der einen gepulsten Laserstrahl 2 emittiert. Die Wellenlänge des Laserstrahls 2 kann Ultraviolett oder Infrarot sein oder aus dem VIS-Spektrum stammen, vorzugsweise liegt sie zwischen etwa 300 und 1100 nm. In einer besonders bevorzugten Ausgestaltung der Erfindung ist der Laser 1 ein UV-Laser mit einer Wellenlänge zwischen 300 und 400 nm.

Im Stand der Technik wird der gepulste Laserstrahl 2 auf eine Ablenkeinheit 20 (Scanner) geführt, die den Laserstrahl 2 in eine vorbestimmte Richtung ablenkt. Der abgelenkte Laserstrahl wird durch ein Aufweitungsteleskop 21 aufgeweitet und durch ein Schneideobjektiv 22 in eine Probe aus transparentem Material 23 fokussiert. Der Laserfokus liegt dabei in einem vorbestimmten Punkt auf einer vorbestimmten Schnittlinie oder -fläche 24. Eine Steuerungseinheit 30 kann die Laserquelle 1 und die Ablenkeinheit 20 ansteuern, um Bestrahlungsparameter zu ändern und/oder um insbesondere einen anderen Ort des Laserfokus (Spot) auf der Schnittlinie oder -fläche 24 vorzubestimmen. Die Steuerungseinheit 30 arbeitet üblicherweise programmgesteuert, d.h. sie umfasst gewöhnlich eine programmierbare Recheneinheit, beispielsweise einen PC.

Neu gegenüber dem Stand der Technik sind die in den Strahlengang des gepulsten Laserstrahls 2 ein- und ausbringbaren Mittel zur Modenkonversion 3, bei deren Durchgang der gepulste Laserstrahl 2 in einen gepulsten Laserstrahl mit helikaler Phasenfront 5 konvertiert wird. Als Mittel zur Modenkonversion eignen sich helikale Phasenplatten, wobei für Hochleistungsanwendungen derzeit segmentierte helikale Phasenplatten zur Verfügung stehen, die aus doppelbrechenden Segmenten mit unterschiedlich orientierten optischen Achsen zusammengesetzt sind (vgl. Fig. 2).

Es ist für die Erfindung nicht zwingend erforderlich, aber bei der Nutzung segmentierter Phasenplatten sehr vorteilhaft, hinter der Phasenplatte einen Raumfilter (oder auch: Raumfrequenzfilter) 4 im Strahlengang anzuordnen. Vor allem das an den Segmentgrenzen unerwünscht gestreute Laserlicht wird so aus dem gepulsten helikalen Laserstrahl 5 entfernt. Die Raumfilterung zur "Strahlreinigung" ist dem Fachmann der Lasertechnik an sich bekannt.

Gemäß der Fig. 5 a) werden die Mittel zur Modenkonversion 3 und der Raumfilter 4 gemeinsam durch Verschieben in den Strahlengang ein- oder ausgeschwenkt, was durch den vertikalen Doppelpfeil angedeutet ist. Der Schwenkvorgang umfasst dabei jede Art der mechanischen Bewegung der Mittel zur Modenkonversion 3 und des Raumfilters 4, die in wenigstens einer Endposition zu einer koaxialen Ausrichtung mit der Laserstrahlrichtung führt. Werden die Mittel zur Modenkonversion 3 und der Raumfilter 4 in die besagte Endposition verbracht (in den Strahlengang eingeschwenkt), dann erfolgt die Umwandlung des gepulsten Laserstrahls 2 in einen gepulsten Laserstrahl mit helikaler Phasenfront 5. Nach dem Ausbringen der Komponenten 3 und 4 steht erneut der Laserstrahl 2 anstelle von 5 zur Verfügung.

Mechanisch ein- und ausbringbare Mittel zur Modenkonversion 3 und Raumfilter 4 sind technisch einfach und kostengünstig, aber nicht unbedingt für den schnellen Wechsel von normalem Laserstrahl 2 zu helikalem Laserstrahl 5 oder umgekehrt in kurzen Zeitintervallen geeignet.

Alternativ ist in Fig. 5 b) daher dargestellt, dass anstelle der nunmehr fixierten Mittel zur Modenkonversion 3 und des ebenfalls fixierten Raumfilters 4 der Strahlengang des Laserlichts 2 über Spiegel 6, 7, 8, 9 umgelenkt wird. Dabei sind die Spiegel 7 und 8 fixiert, und die Spiegel 6 und 9 sind schwenkbar oder ausklappbar. Vermöge einer - nicht dargestellten - Ansteuerung kann der Nutzer durch Auswahl der Spiegelstellung der Spiegel 6 und 9 entscheiden, ob der gepulste Laserstrahl 2 die Mittel zur Modenkonversion 3 und den Raumfilter 4 passieren und in den gepulsten helikalen Laserstrahl 5 überführt werden soll, oder ob er an den Mitteln zur Modenkonversion 3 und am Raumfilter 4 vorbeigeführt wird.

Schließlich sei darauf verwiesen, dass die weiter oben genannte Einrichtung zur Ermittlung der Bestrahlungsdosis vorzugsweise als ein Softwaremodul (z.B. auswählbare Unterroutine) in der Programmierung der Steuerungseinheit 30 integriert ist. Der Einrichtung stehen dann die Bestrahlungsparameter und die auswählbaren Spotraster bereits vor der Durchführung der Bestrahlung zur Verfügung. Sie kann auf der Basis einer Vorausberechnung der Bestrahlungsdosis einen Dosiswert ausgeben oder Warnanzeigen aktivieren, wenn der errechnete Dosiswert einen vorbestimmten Schwellenwert übersteigt.

In einer bevorzugten Ausgestaltung der Erfindung bewirkt das Softwaremodul, dass bei voreingestellten Laserparametern sehr engmaschige Spotraster, die zu einer über den vorbestimmten Schwellenwert hinausgehenden Bestrahlungsdosis pro Flächeneinheit führen würden, gar nicht mehr auswählbar sind oder nur durch gesonderte Freigabe durch den Nutzer aktiviert werden können.

Bezugszeichenliste

| | |
|---|---|
| 1 | Laser |
| 2 | Gepulster Laserstrahl |
| 3 | Mittel zur Modenkonversion |
| 4 | Raumfilter zur "Strahlreinigung" |
| 5 | Gepulster Laserstrahl mit helikaler Phasenfront |
| 6, 9 | Ausklappbare Spiegel |
| 7, 8 | Spiegel |
| 20 | Ablenkeinheit |
| 21 | Aufweitungsteleskop |
| 22 | Schneideobjektiv |
| 23 | Probe aus transparentem Material, z.B. Kornea |
| 24 | Schnittlinie oder -fläche |
| 30 | Steuerungseinheit |

**Patentansprüche**

1.   Vorrichtung zum Laserschneiden eines transparenten Materials (23), die ausgebildet ist, das Laserlicht (2) in eine Mehrzahl vorbestimmter Spots innerhalb des Materials (23) zu fokussieren, wobei die Spots auf einer vorbestimmten Schnittlinie oder -fläche (24) liegen, die im Wesentlichen senkrecht zur Einstrahlrichtung des Laserlichts (2) verläuft,
     **dadurch gekennzeichnet, dass**
     die Vorrichtung in den Strahlengang des Laserlichts (2) ein- und ausbringbare Mittel zur Modenkonversion (3) in Laserlicht mit helikaler Phasenfront (5) aufweist.

2.   Vorrichtung nach Anspruch 1,
     **dadurch gekennzeichnet, dass**
     die Mittel zur Modenkonversion (3) eine helikale Phasenplatte umfassen.

3.   Vorrichtung nach Anspruch 2,
     **dadurch gekennzeichnet, dass**
     die helikale Phasenplatte aus doppelbrechenden Segmenten mit jeweils unterschiedlich orientierten optischen Achsen zusammengesetzt ist.

4.   Vorrichtung nach einem der vorangehenden Ansprüche,
     **dadurch gekennzeichnet, dass**
     die Mittel zur Modenkonversion (3) in den Strahlengang ein- und ausschwenkbar sind.

5.   Vorrichtung nach einem der vorangehenden Ansprüche,
     **dadurch gekennzeichnet, dass**
     wenigstens ein ansteuerbarer Ablenkspiegel (6, 9) vorgesehen ist, der in Abhängigkeit von seiner Ansteuerung den Strahlengang des Laserlichts (2) durch die Mittel zur Modenkonversion (3) führt oder an diesen vorbei.

6. Vorrichtung nach einem der vorangehenden Ansprüche,
   **dadurch gekennzeichnet, dass**
   im Strahlengang des Laserlichts (2) hinter den Mitteln zur Modenkonversion (3) ein Raumfrequenzfilter (4) angeordnet ist.

7. Vorrichtung nach einem der vorangehenden Ansprüche,
   **dadurch gekennzeichnet, dass**
   die Laserlichtquelle (1) gepulstes ultraviolettes Licht (2) mit einer Wellenlänge zwischen 300 nm und 400 nm emittiert.

8. Vorrichtung nach einem der vorangehenden Ansprüche,
   **dadurch gekennzeichnet, dass**
   eine Einrichtung zur Ermittlung der Bestrahlungsdosis pro Flächeneinheit vorgesehen ist, die die Bestrahlungsparameter der Laserlichtquelle (1) und die durch die Steuereinrichtung (30) der Ablenkeinheit (20) vorbestimmten Spotpositionen erfasst, daraus einen Dosiswert errechnet und diesen ausgibt oder eine Warnanzeige bei Überschreiten eines vorbestimmten Schwellenwertes aktiviert.

9. Verwendung der Vorrichtung nach einem der Ansprüche 1 bis 8 zur Bearbeitung lebenden biologischen Materials.

10. Verwendung der Vorrichtung nach einem der Ansprüche 1 bis 8 zur Lasertherapie des lebenden Auges, insbesondere zur Refraktionskorrektur und/oder zur Laser-Kataraktchirurgie.

Fig. 1 a) (prior art)

Linear

XY

XZ

Fig. 1 b) (prior art)

Azimuthal

Fig. 2 (prior art)

Fig. 3 a)

Fig. 3 b)

Fig. 4 a)

Fig. 4 b)

Fig. 5 a)

Fig. 5 b)

| | Europäisches Patentamt<br>European Patent Office<br>Office européen des brevets | **EUROPÄISCHER TEILRECHERCHENBERICHT**<br>nach Regel 62a und/oder 63 des Europäischen Patent-<br>übereinkommens. Dieser Bericht gilt für das weitere<br>Verfahren als europäischer Recherchenbericht. | **Nummer der Anmeldung**<br>EP 12 18 3091 |
|---|---|---|---|

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| Y | US 2008/243108 A1 (MURAKAMI NAHO [JP] ET AL) 2. Oktober 2008 (2008-10-02)<br>* Absatz [0025] - Absatz [0036]; Abbildungen 1-2,3A-3B, *<br>* Absatz [0045] - Absatz [0048]; Abbildungen 4A-4D *<br>----- | 1-8 | INV.<br>A61F9/008<br>H01S3/00<br>B23K26/06<br>B23K26/38<br>B23K26/40 |
| Y | JUNICHI HAMAZAKI ET AL: "Optical-vortex laser ablation",<br>OPTICS EXPRESS,<br>Bd. 18, Nr. 3,<br>1. Februar 2010 (2010-02-01), Seite 2144,<br>XP055053379,<br>ISSN: 1094-4087, DOI: 10.1364/OE.18.002144<br>* das ganze Dokument *<br>-----<br>-/-- | 1-8 | |

**RECHERCHIERTE SACHGEBIETE (IPC)**

A61F
H01S
B23K

### UNVOLLSTÄNDIGE RECHERCHE

Die Recherchenabteilung ist der Auffassung, daß ein oder mehrere Ansprüche, den Vorschriften des EPÜ nicht entspricht bzw. entsprechen, so daß nur eine Teilrecherche (R.62a, 63) durchgeführt wurde.

Vollständig recherchierte Patentansprüche:

Unvollständig recherchierte Patentansprüche:

Nicht recherchierte Patentansprüche:

Grund für die Beschränkung der Recherche:
Siehe Ergänzungsblatt C

| Recherchenort | Abschlußdatum der Recherche | Prüfer |
|---|---|---|
| München | 20. März 2013 | De Backer, Tom |

| KATEGORIE DER GENANNTEN DOKUMENTE | |
|---|---|
| X : von besonderer Bedeutung allein betrachtet<br>Y : von besonderer Bedeutung in Verbindung mit einer anderen Veröffentlichung derselben Kategorie<br>A : technologischer Hintergrund<br>O : nichtschriftliche Offenbarung<br>P : Zwischenliteratur | T : der Erfindung zugrunde liegende Theorien oder Grundsätze<br>E : älteres Patentdokument, das jedoch erst am oder nach dem Anmeldedatum veröffentlicht worden ist<br>D : in der Anmeldung angeführtes Dokument<br>L : aus anderen Gründen angeführtes Dokument<br>.................................................................<br>& : Mitglied der gleichen Patentfamilie, übereinstimmendes Dokument |

EPO FORM 1503 03.82 (P04E09)

Europäisches
Patentamt

European
Patent Office

Office européen
des brevets

**EUROPÄISCHER
TEILRECHERCHENBERICHT**

Nummer der Anmeldung

EP 12 18 3091

| | EINSCHLÄGIGE DOKUMENTE | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| A | BEIJERSBERGEN M W ET AL: "Helical-wavefront laser beams produced with a spiral phaseplate", OPTICS COMMUNICATIONS, NORTH-HOLLAND PUBLISHING CO. AMSTERDAM, NL, Bd. 112, Nr. 5-6, 1. Dezember 1994 (1994-12-01), Seiten 321-327, XP024490043, ISSN: 0030-4018, DOI: 10.1016/0030-4018(94)90638-6 [gefunden am 1994-12-01] * das ganze Dokument * ----- | 1-8 | |
| A,D | US 2005/245915 A1 (LOESEL FRIEDER [DE] ET AL) 3. November 2005 (2005-11-03) * Absatz [0020] - Absatz [0034]; Abbildungen 1,3 * ----- | 1-8 | RECHERCHIERTE SACHGEBIETE (IPC) |
| A | XIANG HAO ET AL: "Effects of polarization on the de-excitation dark focal spot in STED microscopy;Effects of polarization on the de-excitation dark focal spot in STED microscopy", JOURNAL OF OPTICS, INSTITUTE OF PHYSICS PUBLISHING, BRISTOL GB, Bd. 12, Nr. 11, 29. Oktober 2010 (2010-10-29), Seite 115707, XP020200948, ISSN: 2040-8986, DOI: 10.1088/2040-8978/12/11/115707 * das ganze Dokument * ----- | 1-8 | |

EPO FORM 1503 03.82 (P04C12)

Europäisches
Patentamt

European
Patent Office

Office européen
des brevets

**UNVOLLSTÄNDIGE RECHERCHE
ERGÄNZUNGSBLATT C**

Vollständig recherchierbare Ansprüche:
    1-8

Nicht recherchierte Ansprüche:
    9, 10

Grund für die Beschränkung der Recherche (nicht patentfähige Erfindung(en)):

Die "Verwendungs"-Ansprüche 9 und 10 für die "Verwendung der Vorrichtung nach einem der Ansprüche 1 bis 8 zur Bearbeitung lebenden biologischen Materials/ zur Lasertherapie des lebenden Auges, insbesondere zur Refraktionskorrektur und/oder zur Laser-Kataraktchirurgie" werden einem "Verfahrens"-Anspruch für "ein Verfahren zur verwenden der Vorrichtung nach einem der Ansprüche 1 bis 8 zur Bearbeitung lebenden biologischen Materials/ zur Lasertherapie des lebenden Auges, insbesondere zur Refraktionskorrektur und/oder zur Laser-Kataraktchirurgie" gleichgestellt.
Eine sinnvolle Recherche auf der Grundlage der Ansprüche 9 und 10 ist daher nicht möglich, da diese Ansprüche auf ein Verfahren zur chirurgischen Behandlung des menschlichen oder tierischen Körpers gerichtet sind. Ein solches Verfahren ist nach Artikel 53 c) EPÜ von der Patentierbarkeit ausgeschlossen.

**ANHANG ZUM EUROPÄISCHEN RECHERCHENBERICHT
ÜBER DIE EUROPÄISCHE PATENTANMELDUNG NR.**

EP 12 18 3091

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-03-2013

| Im Recherchenbericht angeführtes Patentdokument | Datum der Veröffentlichung | Mitglied(er) der Patentfamilie | Datum der Veröffentlichung |
|---|---|---|---|
| US 2008243108 A1 | 02-10-2008 | JP 5028124 B2<br>JP 2008245833 A<br>US 2008243108 A1 | 19-09-2012<br>16-10-2008<br>02-10-2008 |
| US 2005245915 A1 | 03-11-2005 | EP 1591087 A1<br>JP 4704763 B2<br>JP 2005312921 A<br>US 2005245915 A1 | 02-11-2005<br>22-06-2011<br>10-11-2005<br>03-11-2005 |

EPO FORM P0461

Für nähere Einzelheiten zu diesem Anhang : siehe Amtsblatt des Europäischen Patentamts, Nr.12/82

**EP 2 705 812 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1787607 A1
- DE 102007028042 B3
- US 2005245915 A1

**In der Beschreibung aufgeführte Nicht-Patentliteratur**

- **VOGEL A ; LINZ N ; FREIDANK S ; FAUST S ; SCHWED S.** LASIK Flaperzeugung mit UV Subnanosekundenpulsen. *Der Augenspiegel,* 2011, vol. 12, 32-35
- **MCGLOIN D ; DHOLAKIA K.** Bessel beams: Diffraction in a new light. *Contemp. Phys.,* 2005, vol. 46, 15-28
- **ASHKENASI D ; VAREL H ; ROSENFELD A ; HENZ S ; HERRMANN J ; CAMBELL EEB.** Application of self-focusing of ps laser pulses for three-dimensional microstructuring of transparent materials. *Appl. Phys. Lett.,* 1998, vol. 72, 1442-1444
- **ARNOLD CL ; HEISTERKAMP A ; ERTMER W ; LUBATSCHOWSKI H.** Streak formation as side effect of optical breakdown during processing the bulk of transparent Kerr media with ultra-short laser pulses. *Appl. Phys. B,* 2004, vol. 80, 247-253
- **VUONG LT et al.** Collapse of optical vortices. *Phys. Rev. Lett,* 2006, vol. 96, 133901
- **YAO ; PADGETT.** Orbital angular momentum: origins, behavior and applications. *Advances in Optics and Photonics,* 2011, vol. 3, 161-204
- **MACHAVARIANI G ; LUMER Y ; MOSHE I ; MEIR A ; JACKEL S.** Efficient extracavity generation of radially and azimuthally polarized laser beams. *Opt. Lett.,* 2007, vol. 32, 1468-1470
- **HAO X ; KUANG C ; WANG T ; LIUX.** Effects of polarization on the de-excitation dark focal spot in STED microscopy. *J. Opt.,* 2010, vol. 12, 115707